# EUROPEAN PATENT APPLICATION

(11) **EP 4 242 311 A1**
(43) Date of publication of application: **13.09.2023**
(21) Application number: 21889324.6
(22) Date of filing: 30.04.2021
(51) Int. Cl.: C12N 15/70, C12N 9/02, C09B 7/00, D06P 1/22

(54) **PRODUCTION OF TYRIAN PURPLE CELL DYE BY USING ESCHERICHIA COLI AND DYEING METHOD USING SAME**

(30) Priority: 04.11.2020 KR 20200146303
(71) Applicant: Seoul National University R & DB Foundation, Seoul 08826 (KR)
(72) Inventor: LEE, Jeong Chan, Seoul 08744 (KR); KIM, Byung Gee, Seoul 06587 (KR); KIM, Joon Won, Seoul 04368 (KR)
(74) Representative: Cabinet Becker et Associés
(86) International application number: PCT/KR2021/005504
(87) International publication number: WO 2022/097856

(57) **Abstract**

Tyrian purple, composed primarily of 6,6'-dibromoindigo (6BrIG), is an ancient dye extracted from sea snails that has recently been demonstrated as a biocompatible semiconductor material. However, the synthesis of Tyrian purple is limited due to its unknown biosynthetic pathway and the difficulty of regiospecific bromination. The present invention presents an effective 6BrIG production strategy in *Escherichia coli* using tryptophan 6-halogenase (SttH), tryptophanase (TnaA), and flavin-containing monooxygenase (MaFMO). Since tryptophan halogenase is expressed in a very insoluble form in *Escherichia coli,* flavin reductase Fre, which regenerates FADH₂, was used as an N-terminal soluble tag of SttH for the halogenase reaction. The consecutive two-cell reaction system of the present invention is designed to enable mass production of a regiospecific brominated precursor of 6BrIG by separating time and space for bromination and bromo-tryptophan decomposition. Through this approach, in the present invention, 315.0 mg/L of 6BrIG was produced from tryptophan and successful synthesis of regiospecific dihalogenated indigo was induced. In addition, the present invention suggests that 6BrIG-containing *Escherichia coli* cells can be directly used as a cell dye.

## Description

### [TECHNICAL FIELD]

The present invention relates to a method for preparing halogenated indigo using a recombinant microbial strain, production of a cell dye comprising halogenated indigo, and a dyeing method using the same.

### [BACKGROUND ART]

Tyrian purple, also known as royal purple, is a natural purple dye extracted from Mediterranean sea snails and has recently been studied as a biocompatible semiconductor material. The major component of Tyrian purple is 6,6'-dibromoindigo (hereinafter abbreviated as 6BrIG), and it is known that the purple color of 6BrIG comes from the changes in chromophore of the indigo moiety by two bromine atoms. However, it is very difficult to obtain this dye in large quantities from natural sources as it requires sacrificing 12,000 snails for 1.4 g of the dye.

Due to its scarcity and vibrant color, Tyrian purple was worth more than gold and symbolized the social status of the upper classes in the ancient Roman Empire, and is still an expensive dye today. This dye has orderly stacked and repeated conjugated π bonds and thus can be applied to dye-sensitized solar cells and conductive materials. In addition, various indigo polymers can be synthesized from other monomers derived from 6BrIG by taking advantage of the functional bromine group as a good leaving group.

Nevertheless, it is still difficult to synthesize 6BrIG on an industrial scale. In the case of biosynthesis from sea snails, the genes and enzymes responsible for the synthesis of the intermediates of 6BrIG in sea snails have not yet been fully identified yet, which leads to difficulties. In the case of chemical synthesis, there is still no known productive method for industrialization. According to the chemical synthesis methods reported so far, the problem of the chemical synthesis methods arises from the following difficulties occurring in the process of introducing two bromine atoms in 6BrIG. First, while most of the 6BrIG synthesis methods use bromine gas or hydrogen bromide for bromination, there is a problem that these processes lack regiospecificity, have low product yields, and are environmentally toxic manufacturing processes. Second, when the synthesis begins from brominated precursors unlike the above, the cost of the precursors serves as an obstacle for large-scale synthesis. Thus, the bromination process remains inefficient and uneconomical to be implemented on an industrial scale in both biological and chemical synthesis processes for 6BrIG.

In order to overcome the difficulties in the regiospecific bromination, biocatalysts such as halogenases were proposed as a sustainable method. In this regard, flavin-dependent tryptophan halogenases were introduced as promising biocatalysts for the synthesis of halogenated substances due to its high regiospecificity. Flavin-dependent tryptophan halogenases use halide ion (X⁻) and FADH₂ for halogenation, and were reported to be present in a variety of organisms. Flavin-dependent tryptophan halogenases are classified according to the position of the halogenated carbon in the indole ring; for example, tryptophan 6-halogenase can generate 6-bromotryptophan from tryptophan. However, most studies related to halogenases to date have been carried out using cell-free systems that require supplementation of cofactor FADH₂ and cofactor regenerating enzymes. The high cost of the cofactors and the cofactor regenerating enzymes is regarded as an obstacle to implementation of halogenation on an industrial scale. In order to cope with the economic problem of the cell-free system halogenation, the development of a whole-cell system overexpressing halogenase can be a reasonable alternative for reducing costs and achieving high product yield and productivity.

As a whole-cell system, *Escherichia coli* has advantageous properties as a production host, such as a short cell culture time, the presence of various molecular biological tools for high-efficiency production, and the ability to synthesize and regenerate cofactors such as NAD(P)H or FADH₂. However, the expression of halogenase in *Escherichia coli* has a problem in that it shows low solubility and requires additional use of chaperone for soluble expression. Although there are many reports on halogenase enzyme engineering for increasing the activity of halogenase and changing the regiospecificity, the issue of insoluble expression of halogenase has not been solved. Therefore, additional strategies to improve the solubility of halogenase *in vivo* that is compatible with whole-cell reactions or metabolic engineering are required in order to increase the halogenase activity as well as the yield of target molecules.

Compared to several reports on the biological production of indigo itself in *Escherichia coli,* there are few reports on the biosynthesis of indigoid dyes. It is known that indigoid dyes with various colors can be produced by feeding an appropriate halogenated indole as a substrate to oxygenases such as CYP102G4, naphthalene dioxygenase, and toluene dioxygenase (NON-PATENT DOCUMENTS 1 to 4). However, these documents are limited to showing a proof of concept by using unnatural substrates for indigoid dye production and fail to demonstrate the application of biologically synthesized indigoid dyes in fabric dyeing. In addition, the production of 6BrIG from natural substrates through microbial fermentation has not been reported so far.

On the other hand, cell dyes are recently attracting attention as an alternative to chemically synthesized fiber dyes because they can dramatically reduce toxic waste and byproducts generated in the chemical synthesis process. However, most reports are limited to the use of natural pigments produced by microorganisms in nature.

### [PRIOR ART DOCUMENTS]

### [NON-PATENT DOCUMENTS]

(NON-PATENT DOCUMENT 1) Kim, J.Y. et al. In vitro characterization of CYP102G4 from Streptomyces cattleya: A self-sufficient P450 naturally producing indigo. Biochim Biophys Acta Proteins Proteom 1866, 60-67 (2018)
(NON-PATENT DOCUMENT 2) Zhang, X.W. et al. Cloning and expression of naphthalene dioxygenase genes from Comamonas sp MQ for indigoids production. Process Biochem 48, 581-587 (2013)
(NON-PATENT DOCUMENT 3) Kim, J.Y. et al. Production of dyestuffs from indole derivatives by naphthalene dioxygenase and toluene dioxygenase. Lett Appl Microbiol 36, 343-348 (2003)
(NON-PATENT DOCUMENT 4) Namgung, S. et al. Ecofriendly one-pot biosynthesis of indigo derivative dyes using CYP102G4 and PrnA halogenase. Dyes Pigments 162, 80-88 (2019)

### [DETAILED DESCRIPTION OF THE INVENTION]

### [PROBLEM TO BE SOLVED]

An object of the present invention is to provide a method for preparing halogenated indigo using a recombinant microbial strain, production of a cell dye comprising halogenated indigo, and a dyeing method using the same.

### [MEANS FOR SOLVING THE PROBLEM]

Here, the inventors of the present invention were able to prepare halogenated indigo and a cell dye comprising the same with high efficiency by constructing a biosynthetic pathway for the production of Tyrian purple from tryptophan in *Escherichia coli* and a consecutive two-cell reaction system for selective synthesis of halogenated indigo. In addition, in order to solve the problem of insoluble expression of tryptophan halogenase in *Escherichia coli,* a fusion enzyme of tryptophan halogenase and flavin reductase was used and it further increased the production efficiency of halogenated indigo and a cell dye comprising the same.

Specifically, the present invention provides the following.
(1) A method for preparing halogenated indigo, comprising the steps of:
   a) reacting a recombinant strain expressing tryptophan halogenase with tryptophan; and,
   b) adding a recombinant strain expressing tryptophanse and flavin-containing monooxygenase (FMO) to the reaction mixture of step a) to be reacted.
(2) The method of (1),
   further comprising step a-2) of removing the recombinant strain expressing tryptophan halogenase between steps a) and b).
(3) The method of (1),
   wherein the tryptophan halogenase is tryptophan 5-halogenase, tryptophan 6-halogenase or tryptophan 7-halogenase.
(4) The method of (3),
   wherein the tryptophan halogenase is tryptophan 6-halogenase.
(5) The method of (1),
   wherein the tryptophan halogenase is fused with flavin reductase or maltose-binding protein.
(6) The method of (5),
   wherein the fusion is made at the N-terminus of the tryptophan halogenase.
(7) The method of (5),
   wherein the fusion is made via a linker.
(8) The method of (1),
   wherein the tryptophan halogenase is derived from *Streptomyces toxytricini.*
(9) The method of (1), wherein the flavin-containing monooxygenase is derived from *Methylophaga aminisulfidivorans.*
(10) The method of (1),
   wherein the recombinant strain is *Escherichia coli.*
(11) The method of (1),
   wherein the recombinant strain is a strain in which an endogenous tryptophanase gene is deleted.
(12) The method of (1),
   wherein the halogenated indigo is 5,5'-dihalogenated indigo, 6,6'-dihalogenated indigo or 7,7'-dihalogenated indigo.
(13) The method of (12),
   wherein the halogenated indigo is 6,6'-dibromoindigo.
(14) The method of (1),
   characterized in that each of the reactions of step a) and step b) is performed for 1 to 60 hours at a temperature of 20 to 40° C.
(15) A composition for dyeing fabric, comprising the recombinant strain obtained after the reaction of step b) of (1) is completed as an active ingredient.
(16) The composition of (15),
   wherein the recombinant strain is a lyophilized one.
(17) The composition of (15),
   wherein the fabric is silk or wool.
(18) A method for dyeing fabric, comprising a step of soaking the fabric in the composition of any one of (15) to (17).

### [EFFECT OF THE INVENTION]

Recently, indigo production through various biological routes has been attempted due to environmental concerns, but these processes are not economical enough to replace current chemical synthesis methods. On an industrial scale, indigo is synthesized from aniline, formaldehyde, and hydrogen cyanide, with sodium amide under strong base environment. Through this toxic chemical process, 50,000 tons of indigo are synthesized annually. Unlike the synthesis of indigo, the chemical synthesis of Tyrian purple is not only harmful but also impractical due to the difficulty in synthesizing a regiospecific brominated precursor.

Here, the present invention presents a new production process of Tyrian purple by applying a consecutive two-cell reaction system using tryptophan halogenase, tryptophanase and monooxygenase in *Escherichia coli,* and increases the production efficiency of Tyrian purple by performing the reaction in an optimal order. The present invention is the first invention concerning the production of ancient dye Tyrian purple based on microbial production.

In addition, a dihalogenated indigoid dye produced in the recombinant *Escherichia coli* system described above may be used as a cell dye. In the present invention, non-natural indigo derivatives of various colors such as red, purple, and various blues could be synthesized using naturally derived tryptophan. Previous studies have shown that indole derivatives of various colors can be synthesized from indole derivatives. Further development of enzymatic derivatization on the indole ring such as nitration/amination/methylation/hydroxylation at different positions of the indole ring in order to generate green or yellow can offer more color options for indigo derivatives. In this case, indigo dyes with wider palette ranges can be effectively generated by combining indigo derivatives displaying the three primary colors.

### [BRIEF DESCRIPTION OF THE DRAWINGS]

Fig. 1 shows a biosynthetic pathway of *Escherichia coli* for producing Tyrian purple from tryptophan.
Fig. 2 is a schematic diagram showing fusion enzymes of flavin reductase (hereinafter abbreviated as Fre) and tryptophan 6-halogenase (hereinafter abbreviated as SttH) used in the examples herein.
Fig. 3 shows soluble expression of the fusion enzymes of Fre and SttH confirmed by SDS-PAGE analysis.
Fig. 4 shows that the yield of soluble protein production of the fusion enzymes of Fre and SttH (Fre-L1-SttH, Fre-L2-SttH, and Fre-L3-SttH) increases compared to when SttH is expressed alone.
Fig. 5 shows that, in a whole-cell reaction using *Escherichia coli,* the reactivity of the fusion enzymes of Fre and SttH (Fre-L1-SttH, Fre-L2-SttH, and Fre-L3-SttH) increases compared to when SttH is expressed alone or when SttH and Fre are co-expressed (SttH + Fre).
Fig. 6 shows the amount of Tyrian purple produced through the *Escherichia coli* strain used in the examples herein.
Fig. 7 shows a result of dyeing several fibers using the *Escherichia coli* cell dyes prepared in the examples herein.
Fig. 8 shows result of comparing the dyeing efficiency (K/S value) between dyeing using Tyrian purple cell dyes prepared in the examples herein and dying using chemically synthesized Tyrian purple.
Fig. 9 shows a result of dyeing wool fibers using a cell dye produced on a 1 L reaction scale.
Fig. 10 shows an SDS-PAGE result confirming that tryptophan halogenases (Th-Hal, PyrH, and RebH), which could not be used in *Escherichia coli* cells due to their conventional insoluble expression, can be expressed soluble by constructing a fusion enzyme with Fre.
Fig. 11 is a graph showing the improvement in reactivity of tryptophan halogenase fused with Fre.
Fig. 12 shows a result of the production of six dihalogenated indigo dyes based on the strategy used in the examples herein and the amount of production thereof.

### [DETAILED DESCRITPION TO CARRY OUT THE INVENTION]

The present invention is described in further detail as follows. However, the present invention may be implemented in various modified forms, and is not limited to the embodiments described herein.

Terms used herein are the same as those commonly used in the art unless otherwise defined. In the case of a general term, it should be interpreted as having a meaning consistent with the meaning in the context of the related technology, and should not be interpreted ideally or excessively.

An embodiment of the present invention relates to a method for preparing halogenated indigo using a recombinant microbial strain, comprising the steps of:
a) reacting a recombinant strain expressing tryptophan halogenase with tryptophan; and,
b) adding a recombinant strain expressing tryptophanse and flavin-containing monooxygenase (FMO) to the reaction mixture of step a) to be reacted.

In addition, the method for preparing halogenated indigo according to the present invention may further comprise step a-2) of removing the recombinant strain expressing tryptophan halogenase between steps a) and b).

The tryptophan halogenase according to the present invention collectively refers to enzymes that halogenate tryptophan, and includes tryptophan 5-halogenase, tryptophan 6-halogenase or tryptophan 7-halogenase. Preferably, tryptophan 6-halogenase may be used.

The tryptophan halogenase according to the present invention includes all tryptophan halogenases known in the art, and is derived preferably from the genus Streptomyces, more preferably from *Streptomyces toxytricini,* but is not limited thereto.

In addition, the tryptophan halogenase according to the present invention may be fused with flavin reductase or maltose-binding protein, preferably with flavin reductase, for soluble expression in microbes.

The fusion may be made at the N-terminus or C-terminus of the tryptophan halogenase, preferably at the N-terminus. In addition, the fusion may be made via a linker.

All linkers known to be used for protein fusion in the art may be used as the linker, and the linker includes both a flexible linker and a rigid linker.

The flavin-containing monooxygenase according to the present invention collectively refers to monooxygenases containing FAD as a prothetic group, and includes all flavin-containing monooxygenases known in the art. Preferably, the flavin-containing monooxygenase is derived from the genus Methylophaga, more preferably from *Methylophaga aminisulfidivorans,* but is not limited thereto.

The recombinant strain according to the present invention includes all microbial strains used for the expression of a recombinant protein in the art, and is preferably *Escherichia coli,* but is not limited thereto.

In addition, a strain in which an endogenous tryptophanase gene is deleted may be used as the recombinant strain in order to prevent the influence of expression of endogenous tryptophanase. For example, an *Escherichia coli* strain in which the endogenous tnaA gene is deleted may be used.

The indigoid dye according to the present invention refers to a dye comprising an indigo derivative, and includes halogenated indigo. The halogenated indigo according to the present invention includes dihalogenated indigo such as 5,5'-dihalogenated indigo, 6,6'-dihalogenated indigo, or 7,7'-dihalogenated indigo, and is preferably 6,6'-dihalogenated indigo, more preferably 6,6'-dibromoindigo.

The reactions in step a) and step b) are carried out under culture conditions for microbial strains known in the art. For example, the reactions may be performed at a temperature range of 4 to 40 °C, preferably at a temperature range of 20 to 40 °C, more preferably at 30 to 37 °C. The reaction time may be 1 to 60 hours, but is not limited thereto.

Another embodiment of the present invention relates to a composition for dyeing fabric, comprising the recombinant strain obtained after the reaction of step b) is completed as an active ingredient. The recombinant strain is characterized in that the halogenated indigo produced from the reaction of step b) is contained in a cell. Preferably, the recombinant strain is included in the composition for dyeing fabric after being lyophilized.

The composition for dyeing fabric according to the present invention may be used for dyeing all fabrics known in the art. For example, it may be used for dyeing fabrics such as silk, acryl, rayon, wool, acetate, nylon, and cotton. Preferably, it is suitable for dyeing animal fibers such as wool and silk.

Another embodiment of the present invention relates to a method for dyeing fabric, comprising a step of soaking the fabric in the composition for dyeing fabric.

The present invention is described in further detail through the following examples. Various modifications may be made to the examples described below. The examples described below are not intended to be limited to the embodiments, and should be understood to include all modifications and substitutions thereof. Terms used in the examples are not intended to limit specific situations, but are intended to describe the examples.

### [Examples]

### Example 1: Construction of 6-bromoindole production pathway reaction system from tryptophan

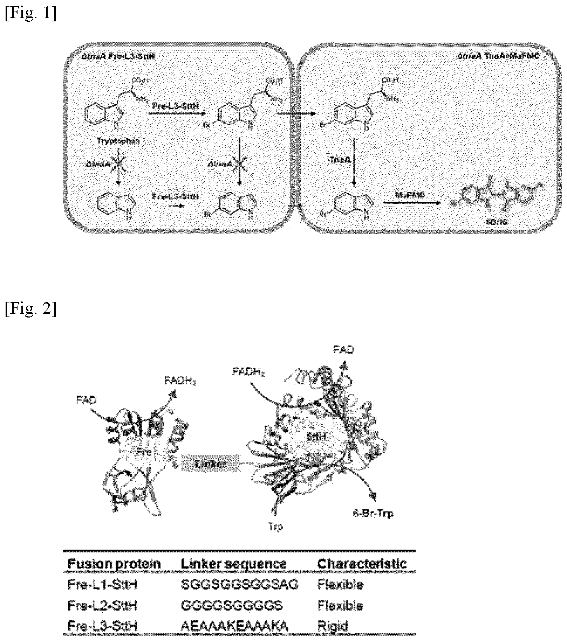

Tryptophan was used as a starting material for the sustainable production of 6BrIG. Based on previous studies on the biological synthesis of 6BrIG from 6-bromoindole, focus was put on the overproduction of 6-bromoindole from tryptophan. 6-bromoindole can be synthesized by two pathways using SttH and TnaA: tryptophan is first converted to indole, which is then brominated, or tryptophan is brominated and then converted to 6-bromoindole (Fig. 1). In order to validate the two pathways, all enzymatic steps using tryptophanase (TnaA) derived from *Escherichia coli* or tryptophan halogenase (SttH) derived from *Streptomyces toxytricini* were evaluated individually through whole-cell reactions. An *Escherichia coli* strain in which the tnaA gene was deleted was used in order to prevent the influence of expression of endogenous TnaA, and TnaA was overexpressed using a plasmid when necessary throughout this study.

First, TnaA activity for tryptophan and 6-bromotryptophan was evaluated. TnaA consumed 1 mM of the substrate within 2 hours at a similar consumption rate, which indicates that TnaA is effective enough to convert tryptophan and 6-bromotryptophan to the corresponding indole. Next, the reaction between SttH and indole or tryptophan was evaluated. Considering that halogenating enzymes sometimes show altered regiospecificity for different substrates, the regiospecificity of SttH for tryptophan and indole was evaluated through an *in vitro* reaction. SttH produced only 6-bromotryptophan from tryptophan as previously reported. Even in the case of indole, SttH showed strict regiospecificity for both bromination and chlorination at the C6 position. Thus, SttH was suitable for C6 regiospecific bromination of the indole ring from tryptophan or indole. How SttH reacted with indole and tryptophan was confirmed using a whole-cell reaction. Since SttH showed low reactivity when expressed alone, SttH was expressed together with Fre for regeneration of FADH₂ for SttH reaction. When 2.5 mM of indole or tryptophan was used as a substrate, 0.29 mM of 6-bromoindole or 0.78 mM of 6-bromotryptophan was produced. In conclusion, SttH exhibited about 2.5-fold higher activity for tryptophan than indole. To summarize, the activity data suggest that SttH reaction followed by TnaA reaction (tryptophan → 6-bromotryptophan → 6-bromoindole) can give a higher yield of 6-bromoindole, and would be advantageous for 6BrIG production compared to the other pathway (tryptophan → indole → 6-bromoindole).

### Example 2: Preparation of fusion enzyme of tryptophan halogenase and flavin reductase for improving solubility and reactivity of tryptophan halogenase

Although SttH was successfully overexpressed in *Escherichia coli,* it was mostly present in an insoluble form, limiting the yield of bromination in the whole-cell reaction to a maximum of 31.2% (0.78 mM of 6-bromotryptophan). Accordingly, in order to increase the bromination reaction rate, an attempt was made to increase the solubility of SttH by preparing a fusion enzyme in which a soluble protein was fused to the N-terminus of SttH. Fre was chosen as a fusion partner for SttH because it is highly expressed in *Escherichia coli* in a soluble form and can efficiently regenerate FADH₂. Two flexible linkers and one rigid linker were compared, taking into account that a linker may affect substrate diffusion into an active site of an enzyme or that the flexibility of the linker may cause a decrease in activity. To this end, three fusion enzymes were constructed using three linker sequences having different flexibility, and the constructed fusion enzymes were named Fre-L1-SttH, Fre-L2-SttH, and Fre-L3-SttH, respectively (Fig. 2).

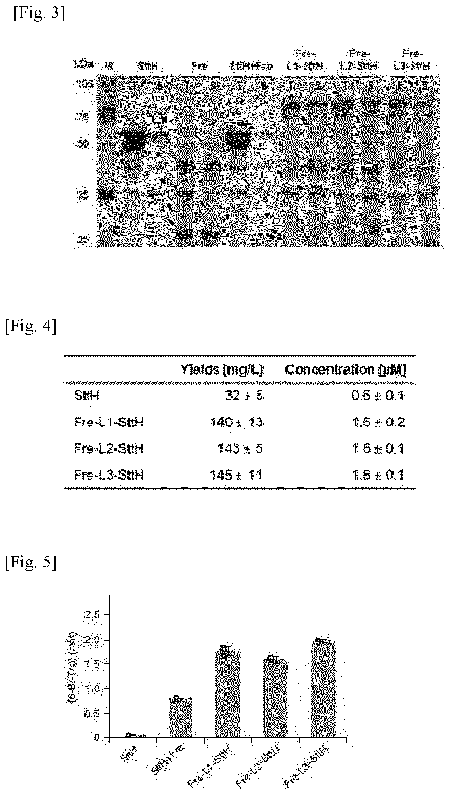

After overexpression of the constructed fusion enzymes in *Escherichia coli,* the expression levels and ratios of the soluble form of SttH were confirmed by SDS-PAGE (Fig. 3). As a result, it was found that the total expression levels of SttH in the fusion enzymes decreased, but the ratios of the soluble form increased. Specifically, the protein yields of Fre-L1-SttH, Fre-L2-SttH, and Fre-L3-SttH were 1.59, 1.62, and 1.64 µM, respectively, which are more than three times higher than the protein yield of solely expressed SttH without fusion (0.53 µM) (Fig. 4). It was confirmed that, although the expression levels of the fused SttH enzymes were similar to that of Fre, they were relatively lower than the total expression level of SttH alone, and the protein expression levels were often affected by the protein N-terminal region. For comparative experiments, a fusion enzyme in which Fre was fused to the C-terminus of SttH was additionally constructed and the expression level thereof was observed, to find that, as in the case of expression of SttH alone, the total expression level was higher, but most of it was present in an insoluble form. In summary, Fre is effective as a soluble N-terminal tag for SttH.

The activities of the fusion enzymes were evaluated by an *in vivo* whole-cell reaction using tryptophan as a substrate. The production of 6-bromotryptophan was compared in an *Escherichia coli* strain in which the tnaA gene was deleted (Δ*tnaA*)*.* A strain expressing SttH alone (Δ*tnaA* SttH), a strain co-expressing SttH and Fre (Δ*tnaA* SttH+Fre), a strain expressing Fre-L1-SttH (Δ*tnaA* Fre-L1-SttH), a strain expressing Fre-L2-SttH (Δ*tnaA* Fre-L2-SttH), and a strain expressing Fre-L3-SttH (Δ*tnccA* Fre-L3-SttH) were cultured in 50 mM of a sodium phosphate buffer containing 2.5 mM of tryptophan, 300 mM of sodium bromide, and 0.6% (w/v) of glucose, respectively, before comparing the amount of 6-bromotryptophan produced. The result was that, in the strain co-expressing SttH and Fre, the amount of 6-bromotryptophan produced (0.78 mM) was 15 times higher than that of the strain expressing SttH alone (0.05 mM) (Fig. 5), indicating that FADH₂ deficiency is a rate-limiting factor. In addition, in the strain expressing Fre-L1-SttH, strain expressing Fre-L2-SttH, and strain expressing Fre-L3-SttH, the productions of 6-bromotryptophan were greatly increased to 1.78, 1.59, and 1.98 mM, respectively (Fig. 5). Especially, the strain expressing Fre-L3-SttH showed the highest amount of 6-bromotryptophan produced, which was 39 times and 2.5 times higher than those of the strain expressing SttH alone and the strain co-expressing SttH and Fre, respectively. This suggests that the fusion strategy used in this example is effective in increasing 6-bromotryptophan production in tryptophan.

In order to confirm whether the increased 6-bromotryptophan production results from the enhaned solubility or the accelerated cofactor regeneration, SttH was fused with maltose binding protein (MBP), one of the well-known soluble tags in the art. A fusion enzyme, MBP-L3-SttH, was produced by fusing MBP and SttH via linker L3, and its expression level was compared with that of Fre-L3-SttH. As a result, it was observed that MBP-L3-SttH showed an increased ratio of soluble forms of SttH as Fre-L3-SttH did. Then, as a result of comparing bromination reactivity through a whole-cell reaction experiment, it was found that the strain expressing MBP-L3-SttH (Δ*tnaA* MBP-L3-SttH) produced 0.07 mM of 6-bromotryptophan, a 1.4-fold increase compared to the strain expressing SttH alone. Considering that the strain co-expressing SttH and Fre produced 15-fold more 6-bromotryptophan than the strain expressing SttH alone, it was confirmed that the fusion with Fre contributes to improvement in 6-bromotryptophan production more than the solubilization effect of SttH. Moreover, when Fre was co-expressed with MBP-L3-SttH (Δ*tnaA* MBP-L3-SttH+Fre), 6-bromotryptophan production increased 19-fold (0.95 mM of 6-bromotryptophan) than when SttH was expressed alone, which confirmed that the two effects were cumulative to a certain extent. However, more interestingly, the activity of the strain expressing Fre-L3-SttH was 2.1-fold higher than that of the strain co-expressing Fre and MBP-L3-SttH, which suggests that, for SttH, Fre is a better soluble tag than MBP. This improvement in productivity is thought to result from the improved electron transfer efficiency through the proximity effect of electron transfer partners caused by the fusion of Fre and SttH. In summary, it is suggested that Fre is a unique N-terminal tag that efficiently enhances SttH activity by increasing both solubility and cofactor supply.

### Example 3: Production of Tyrian purple indigoid dye in Escherichia coli

In previous studies, it was reported that CYP102G4 and flavin-containing monooxygenase derived from *Methylophaga aminisulfidivorans* (hereinafter abbreviated as MaFMO) effectively synthesized indigo by oxidation of indole (Han, G.H. et al. Enzyme Microb Tech 42, 617-623 (2008) and Kim, H.J. et al., Dyes Pigments 140, 29-35 (2017)). Accordingly, in order to produce 6BrIG from 6-bromoindole, a strain co-expressing CYP102G4 and TnaA (Δ*tnaA* TnaA+CYP102G4) and a strain co-expressing MaFMO and TnaA (Δ*tnaA* TnaA+MaFMO) were constructed, and their expression conditions were optimized.

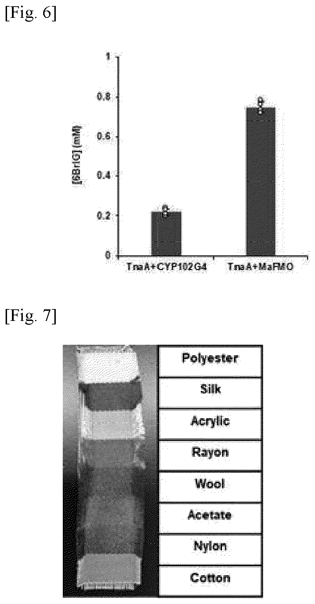

First, after bromination of tryptophan was carried out for 24 hours using a strain expressing Fre-L3-SttH, a strain co-expressing CYP102G4 and TnaA or a strain co-expressing MaFMO and TnaA was added to the supernatant fluid of the reaction mixture, and the concentrations of 6-bromotryptophan, 6-bromoindole, and 6BrIG were observed over the reaction time. As a result, it was confirmed that the reactions using MaFMO and CYP102G4 produced 0.75 mM (315.0 mg/L) and 0.22 mM (92.4 mg/L) of 6BrIG, respectively, after 8 hours of reaction, indicating that MaFMO produced 3.4 times more 6BrIG than CYP102G4 (Fig. 6). Moreover, since a very low concentration of tryptophan (0.09 mM) remained after the bromination step by the strain expressing Fre-L3-SttH, a very small amount of by-product indigo (the theoretical maximum content thereof being less than 0.045 mM) would exist in the final reaction mixture of the strain co-expressing MaFMO and TnaA. Therefore, this suggests that up to 0.75 mM (315 mg/L) of 6BrIG can be regiospecifically produced from 2.5 mM of tryptophan by constructing the biosynthetic pathway in *Escherichia coli* and using the consecutive two-cell reaction system.

### Example 4: Fabric dyeing using Tyrian purple-producing Escherichia coli as a cell dye

The resulting 6BrIG dye was intercalated into cell membranes and often accumulated in cells because the indigoid compound was hydrophobic and no known indigoid secretory transporter was found in *Escherichia coli.* Therefore, it was evaluated whether *Escherichia coli* cell pellets containing biosynthetic dyes could be directly used for dyeing, as in the case of using sea snails containing Tyrian purple. In addition, the dyeing efficiencies of cell dyes and chemical dyes were compared. First, various fabrics were dyed with 6BrIG-containing *Escherichia coli* cell pellets in order to identify fabrics suitable for 6BrIG dyeing. As a result, among all fabrics investigated, animal fibers such as silk and wool were dyed more effectively (Fig. 7).

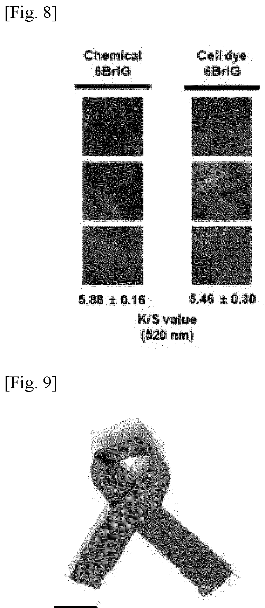

In order to evaluate the dyeing properties of cell pellets in this example, wool fabrics were dyed with a chemical dye as a control group and also with a cell dye prepared to have the same amount of 6BrIG as the chemical dye. In order to evaluate the dyeing efficiency, the K/S value was measured at 520 nm to measure the color depth of the dyed fabric, that is, the dyeing efficiency. As a result, the K/S value of the cell dye 6BrIG was slightly lower than that of the chemical dye 6BrIG (Fig. 8), which explains that the cell dye can have a dyeing efficiency comparable to that of the chemical dye.

In addition, in order to quantitatively compare the brightness and color of the dyed fabrics, a brightness indicator (L*), a red-to-green indicator (a*), and a yellow-to-blue indicator (b^{∗}) were analyzed. All values obtained using the 6BrIG cell dye and the chemical 6BrIG were very similar with low color difference (E^{∗}_{ab}), indicating that the cell dye containing 6BrIG exhibits similar color characteristics to the chemical dye.

Similarly, dyeing characteristics of an indigo cell dye in which Fre-L3-SttH is not expressed were further compared with dyeing characteristics of commercially available indigo. As in the case of 6BrIG, all color indicators obtained by between using chemical indigo and indigo cell dyes are similar, suggesting that both types of cell dyes have similar color characteristics to their chemical counterparts. The indigo cell dyes have a dyeing efficiency of 86.8% compared to the indigo chemical dye, and it could be improved through optimization of the dyeing process in the future. Finally, as a proof-of-concept application using 6BrIG-containing *Escherichia coli* cell pellets, wool gauze was dyed to confirm the natural color of 6BrIG (Fig. 9).

### Example 5: Biosynthesis of various dihalogenated indigoid dyes through the strategy of construction of Escherichia coli consecutive two-cell reaction system and fusion enzyme

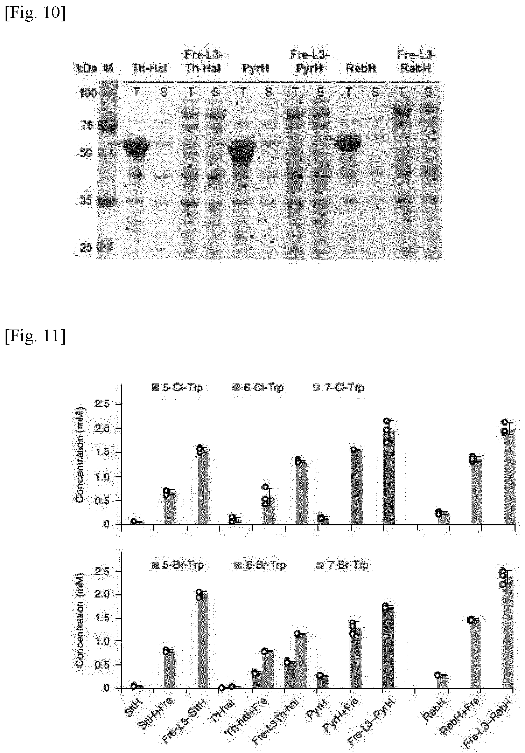

In order to evaluate whether the strategy in the previous examples could be extended to the synthesis of other dihalogenated indigo, the inventors of the present invention applied the fusion strategy and the consecutive two-cell reaction system to other halogenated enzymes. First, using L3 used above as the linker, the fusion strategy was applied to tryptophan 6-halogenase (Th-Hal) derived from *Streptomycin violaceusniger,* tryptophan 5-halogenase (PyrH) derived from *Streptomyces rugosporus,* and tryptophan 7-halogenase (RebH) derived from *Lechevalieria aerocolonigenes* to construct fusion enzymes Fre-L3-Th-Hal and Fre-L3-PyrH, and Fre-L3-RebH, respectively. Wild-type Th-Hal, PyrH, and RebH were mostly expressed as insoluble proteins, but all of the proteins fused with Fre were expressed in soluble forms (Fig. 10). In addition, all halogenation reactions using the Fre-L3-halogenases maintained their regiospecificity and showed improved activity compared to when the halogenase was used alone (Fig. 11). In conclusion, it is suggested that the fusion strategy of the present invention can be generally applied to effectively enhance the performance of tryptophan halogenase for whole-cell reactions.

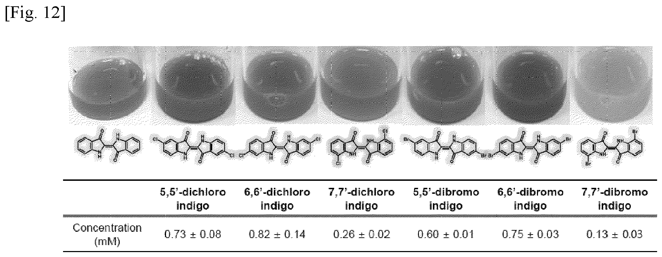

Next, dihalogenated indigo production was carried out by applying the consecutive two-cell reaction system. In order to produce dihalogenated indigo of various colors by altering the type of halogen atom and the halogenation position of the indole ring, combinations of halide salts (NaCl and NaBr) and the fusion enzymes of Fre and halogenase having different regiospecificity (Fre-L3-PyrH, Fre-L3-SttH, Fre-L3-RebH) were applied to the consecutive two-cell reaction system. As a result, based on the premise that halogenated indole can be produced by TnaA reaction with the corresponding halogenated tryptophan, 5,5'-dichloroindigo (0.73 mM), 6,6'-dichloroindigo (0.82 mM), 7,7'-dichloroindigo (0.26 mM), 5,5'-dibromoindigo (0.60 mM), 6,6'-dibromoindigo (0.75 mM), and 7,7'-dibromoindigo (0.13 mM) could be produced from 2.5 mN of tryptophan as an initial substrate (Fig. 12). In conclusion, dihalogenated indigo dyes of various colors can be produced with regiospecificity, demonstrating versatility of the consecutive two-cell reaction system.

### [INDUSTRIAL APPLICABILITY]

The 6BrIG production strategy using the recombinant strain according to the present invention and a cell dye obtained thereby can be applied to a dyeing process for various fabrics, which is expected to have a wide range of future applications in that it is an environmentally friendly process.

## Claims

1. A method for preparing halogenated indigo, comprising the steps of:
a) reacting a recombinant strain expressing tryptophan halogenase with tryptophan; and,
b) adding a recombinant strain expressing tryptophanse and flavin-containing monooxygenase (FMO) to the reaction mixture of step a) to be reacted.

2. The method of claim 1, further comprising step a-2) of removing the recombinant strain expressing tryptophan halogenase between steps a) and b).

3. The method of claim 1, wherein the tryptophan halogenase is tryptophan 5-halogenase, tryptophan 6-halogenase or tryptophan 7-halogenase.

4. The method of claim 3, wherein the tryptophan halogenase is tryptophan 6-halogenase.

5. The method of claim 1, wherein the tryptophan halogenase is fused with flavin reductase or maltose-binding protein.

6. The method of claim 5, wherein the fusion is made at the N-terminus of the tryptophan halogenase.

7. The method of claim 5, wherein the fusion is made via a linker.

8. The method of claim 1, wherein the tryptophan halogenase is derived from *Streptomyces toxytricini.*

9. The method of claim 1, wherein the flavin-containing monooxygenase is derived from *Methylophaga aminisulfidivorans.*

10. The method of claim 1, wherein the recombinant strain is *Escherichia coli.*

11. The method of claim 1, wherein the recombinant strain is a strain in which an endogenous tryptophanase gene is deleted.

12. The method of claim 1, wherein the halogenated indigo is 5,5'-dihalogenated indigo, 6,6'-dihalogenated indigo or 7,7'-dihalogenated indigo.

13. The method of claim 12, wherein the halogenated indigo is 6,6'-dibromoindigo.

14. The method of claim 1, **characterized in that** each of the reactions of step a) and step b) is performed for 1 to 60 hours at a temperature of 20 to 40° C.

15. A composition for dyeing fabric, comprising the recombinant strain obtained after the reaction of step b) of claim 1 is completed as an active ingredient.

16. The composition of claim 15, wherein the recombinant strain is a lyophilized one.

17. The composition of claim 15, wherein the fabric is silk or wool.

18. A method for dyeing fabric, comprising a step of soaking the fabric in the composition of any one of claims 15 to 17.
